# EUROPEAN PATENT APPLICATION

(11) **EP 1 430 929 A1**
(43) Date of publication of application: **23.06.2004**
(21) Application number: 03020463.0
(22) Date of filing: 15.09.2003
(51) Int. Cl.: A61N 1/37

(54) **Heart stimulating device**

(30) Priority: 16.12.2002 SE 0203725
(71) Applicant: St. Jude Medical AB, 175 84 Järfälla (SE)
(72) Inventor: Öhman, Magnus, 165 72 Hässelby (SE)

(57) **Abstract**

Heart stimulating device (2) comprising stimulation threshold measuring means (4) for performing threshold searches by measuring a stimulation threshold value, a pulse generator (6) for delivering stimulation pulses of variable amplitudes and duration, a control means (8) for controlling the pulse generator (6) to deliver stimulation pulses of amplitudes related to the measured threshold value, an interval timing means (10) for initiating time-initiated threshold searches, wherein the time between consecutive time-initiated threshold searches is defined as a threshold search interval. The interval timing means (10) varies said threshold search interval, in dependence of the result of at least the last performed threshold search.

## Description

### Field of the invention

The present invention relates to a heart stimulating device according to the preamble of the independent claim.

### Background of the invention

To reduce the energy consumption of heart stimulators an automatic threshold search function, a so called AUTOCAPTURE™ pacing system function, is provided to maintain the energy of the stimulation pulses at a level just above that which is needed to effectuate capture, cf. e.g. US-A-5,458,623.

A threshold search is a safety measure to ensure that each stimulation pulse leads to capture and the reason for performing a threshold search is that the threshold value, i.e. the smallest amount of energy resulting in depolarisation of the myocardium, is not constant.

A threshold value is often referred to as an amplitude value, i.e. the voltage that the pacemaker stimulates at. In fact, it is the strength and duration of current that reaches the heart muscle cells that determines whether the muscle will contract. The strength and duration is determined by the amplitude, pulse width and the characteristics of the electrode tip, as well as its position in relation to the myocardium.

In one usual implementation of a threshold search algorithm, the pulse width is programmed to a constant value at implantation or follow-up and is not altered by the threshold search function.
When describing the present invention the threshold value is often exemplified as the voltage value but it should be kept in mind that it is also possible to alter the pulse width and having a constant amplitude value.

In the present invention any method to modify the stimulation pulse energy, to determine the threshold value, can be used.

A person skilled in the art of heart stimulators is aware of many different threshold search algorithms. The principles of a preferred threshold search algorithm will only be briefly described. According to the preferred algorithm the AV-interval is shortened in order to override any intrinsic heart activity and the ventricular stimulation amplitude is successively stepped down by a predetermined amplitude step of e.g. 0,3 V.

The threshold value is defined by the AUTOCAPTURE function as the lowest amplitude capable of initiating depolarisation twice in succession. The value can change both upward and downward.

If the threshold value increases, the pacemaker notices it immediately as there is no capture. With evoked response detection, the pacemaker is immediately informed that no capture has occurred. If it happens twice in succession, the pacemaker increases the amplitude by 0,3 volt and it now looks for an amplitude from which it can begin threshold-value detection again. To do this, it needs to identify two captures in succession. If capture occurs every other time, it continues to stimulate at the same amplitude. Every time there are two successive losses, it raises the amplitude.

If the pacemaker has not detected an evoked response after 62,5 ms after the stimulation pulse, it concludes that there was no capture. A back-up safety pulse is then immediately delivered with high energy. The back-up pulse may have an amplitude of 4,5 V and a width of 0,49 ms (compared to a normal stimulation pulse that has a pulse width in the order of 0,2 - 0,3 ms).

On the other hand, if the threshold value falls, the pulse generator will adjust the amplitude accordingly (however not immediately). It measures the threshold value regularly to be able to benefit from the saving of energy that a lower threshold value makes possible. This is done at eight-hour intervals.
When the threshold value is measured, the amplitude is reduced 0,3 volt at a time until it looses capture twice in succession. It then returns to the amplitude 0,3 volt above, to confirm that it really is the threshold value. This is confirmed by two successive captures.

Assume that the threshold value prior to detection was 1,2 volts. In that case, the pacemaker will have stimulated at 1,5 volts. The amplitude is first reduced to 1,2 volts. Two captures in succession confirm that it is not a value below the threshold. The pacemaker then tries an amplitude 0,3 volts lower, i.e. 0,9 volt. If there are two captures at this amplitude as well, the pacemaker continues downwards and tries 0,6 volt. If there are then two successive no captures, it means it has gone below the threshold. The amplitude is raised 0,3 volt again to 0,9 volt. If there are again two captures, it confirms that 0,9 volt is now the threshold value. The new stimulation amplitude is the threshold value plus a working margin, e.g. 0,3 volt, i.e. in this case, 1,2 volts.
Thus, the rule is to seek the lowest amplitude that gives two successive captures and then establish the new stimulation amplitude at the value plus a safety margin of e.g. 0,3 volt.

St Jude Medical AB, the applicant of the present application, provides a heart stimulator with a threshold search capability that uses a threshold search algorithm that works in line with the threshold search algorithm described above and where the time between automatic testing of the stimulation threshold is fixed to 8 hours.

It is well known in the literature and through studies that the stimulation threshold changes during the first month after implantation and that for the following longevity of the device, the stimulation threshold is constant unless there are special events.

It has been found that threshold searches with associated loss of capture and backup stimulation may have pro-arrhythmic effects resulting in patient discomfort due to initiated arrhythmias.

The object of the present invention is to further optimise the threshold search algorithm in order to minimise the potential discomfort for the patient.

### Summary of the invention

The above-mentioned object is achieved by the present invention according to the independent claim.

Preferred embodiments are set forth in the dependent claims.

The above-mentioned object is achieved by the present invention by an automatic variation of the time interval between time-initiated threshold searches. This automatic threshold search interval regulation may be based on the historical data from the result of previous time-initiated threshold searches. The threshold interval regulation may optionally also be based on the result from Loss-of Capture (LOC) initiated threshold searches.

Thus, according to the present invention a variable threshold search interval is introduced.

Minimum and maximum time intervals can be fixed or programmable by the user. Typical values for minimum time can be 8 hours as is presently used in the devices today, and the maximum time 1 month.

The beat to beat threshold search function with back-up pulses ensures that there is always capture and in the event of unexpected threshold rises that the stimulation amplitude is increased to a new value that provides capture stimulations.

The need for the time-initiated threshold searches is to follow a declining threshold so that the stimulation is made with the lowest energy required. If the threshold is constant, the time between threshold searches can therefore be prolonged without negative impact.

### Short description of the appended drawings

Figure 1 shows a simplified block diagram of a heart stimulating device according to the present invention.
Figure 2 shows a flow diagram illustrating the main steps performed by a preferred embodiment of the present invention.

### Detailed description of preferred embodiments of the invention

As indicated in the background section a threshold search may be initiated for two different reasons. One is a time-initiated threshold search and the other is a loss-of capture (LOC) initiated threshold search.

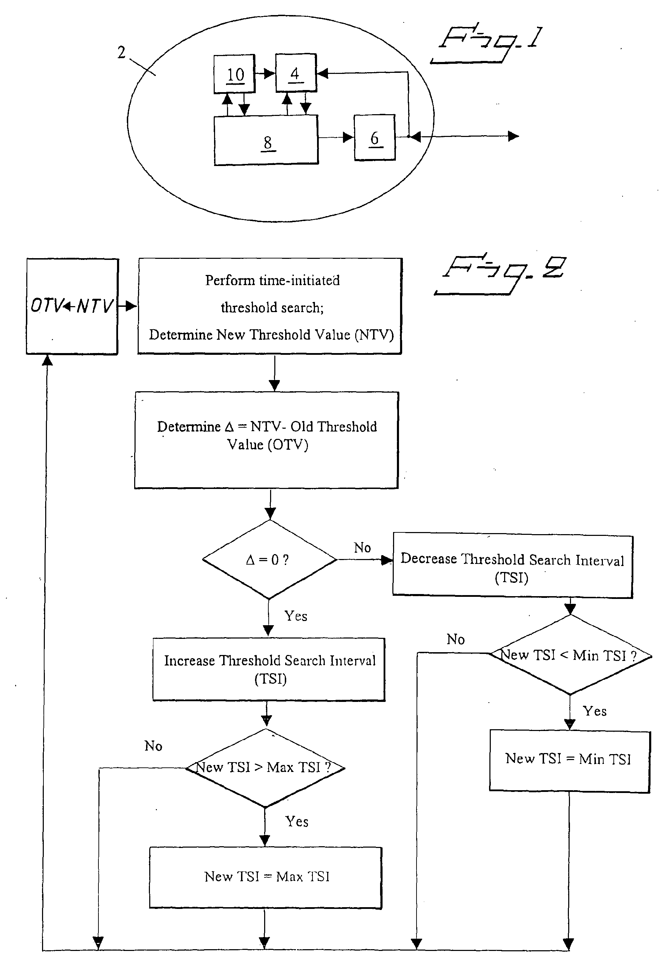

The present invention will now be described by referring to figure 1 that shows a simplified block diagram of a heart stimulating device according to the present invention. In figure 1 is disclosed a heart stimulating device 2 comprising stimulation threshold measuring means 4 for performing threshold searches by measuring a stimulation threshold value, a pulse generator 6 for delivering stimulation pulses of variable amplitudes and duration, a control means 8 for controlling the pulse generator to deliver stimulation pulses of amplitudes related to the measured threshold value, and an interval timing means 10 for initiating time-initiated threshold searches. The time between consecutive time-initiated threshold searches is defined as a threshold search interval.

According to the present invention the interval timing means 10 varies said threshold search interval, in dependence of the result of at least the last performed threshold search.

The threshold value is changed in accordance with the following general rules:
If the threshold value did not change at the last performed threshold search, the threshold search interval is increased.
If the threshold value did change at the last performed threshold search, the threshold search interval is decreased.

In a preferred embodiment of the present invention the threshold search interval is variable by increasing or decreasing the interval length with a predetermined percentage of the threshold search interval. A preferred percentage would be in the range 5-25% of the present threshold search interval.

As an alternative, the threshold search interval could be varied by increasing or decreasing the interval length with a preset time. The preset time is in the range 1 hour - 4 days.

The maximal threshold search interval is preferably one month and the minimum threshold search interval is preferably one hour.

As indicated in the background section a threshold search is also initiated, in addition to the time initiated threshold search, if loss of capture is detected. The thresholds before and after all loss of capture initiated threshold searches between the previous and the present time initiated threshold searches are checked, and the results of these checks are used when varying the threshold search interval length. In this situation the threshold search interval is increased if the thresholds before and after all loss of capture initiated threshold searches are equal.

According to an improvement of the preferred embodiment of the present invention in the case that a threshold change has been made, the threshold search interval is maintained, for a predetermined time period after the threshold change, at a duration shorter or equal than a predetermined threshold search interval.

The predetermined threshold search interval is preferably 8 hours and the predetermined time period is preferably longer or equal one week.

Figure 2 shows a flow diagram illustrating the main steps performed by a preferred embodiment of the present invention.

When the present Threshold Search Interval (TSI) ends, a new time-initiated threshold search is performed, by the stimulation threshold measuring means 4 (in figure 1), and a new Threshold Value (NTV) is determined.
The difference Δ between the NTV and the Old Threshold Value (OTV) is determined by the control means 8 and it is checked if Δ equals 0 or not.
If Δ equals 0, meaning that no threshold change is determined, the TSI is increased by the interval timing means 10.
The New TSI is compared, in the interval timing means 10, to a maximal acceptable TSI and if the new TSI is greater than max TSI the new TSI is set to max TSI.
If Δ not equals 0, meaning that a threshold change is determined, the TSI is decreased by the interval timing means 10.
The new, decreased TSI is compared, in the interval timing means 10, to a minimal acceptable TSI and if the new TSI is smaller than min TSI the new TSI is set to max TSI. As soon as the above procedure has ended the new TSI starts by the interval timing means 10.

Before the next threshold search is due, an Old Threshold Value (OTV) is given the value of the New Threshold Value (NTV).

The flow diagram in figure 2 only shows the main features of the present invention and many of the different embodiments discussed in relation to figure 1 is not shown.

The present invention is not limited to the above-described preferred embodiments. Various alternatives, modifications and equivalents may be used. Therefore, the above embodiments should not be taken as limiting the scope of the invention, which is defined by the appended claims.

## Claims

1. Heart stimulating device (2) comprising stimulation threshold measuring means (4) for performing threshold searches by measuring a stimulation threshold value, a pulse generator (6) for delivering stimulation pulses of variable energy, a control means (8) for controlling the pulse generator to deliver stimulation pulses with energy related to the measured threshold value, an interval timing means (10) for initiating time-initiated threshold searches, wherein the time between consecutive time-initiated threshold searches is defined as a threshold search interval, **characterized in that** said interval timing means varies said threshold search interval, in dependence of the result of at least the last performed threshold search.

2. Heart stimulating device according to claim 1, **characterized in that** if the threshold value did not change at the last performed threshold search, the threshold search interval is increased.

3. Heart stimulating device according to claim 1, **characterized in that** if the threshold value did change at the last performed threshold search, the threshold search interval is decreased.

4. Heart stimulating device according to claim 1, **characterized in that** the threshold search interval is variable by increasing or decreasing the interval length with a preset time.

5. Heart stimulating device according to claim 1, **characterized in that** the threshold search interval is variable by increasing or decreasing the interval length with a predetermined percentage of the actual threshold search interval.

6. Heart stimulating device according to claim 1, **characterized in that** the maximal threshold search interval is one month.

7. Heart stimulating device according to claim 1, **characterized in that** the minimum threshold search interval is one hour.

8. Heart stimulating device according to claim 1, **characterized in that** the threshold values before and after all loss of capture initiated threshold searches, during the present threshold search interval, are used in addition to time initiated threshold searches when determining the threshold search interval length.

9. Heart stimulating device according to claim 8, **characterized in that** the threshold search interval is increased if the thresholds before and after all loss of capture initiated threshold searches are equal.

10. Heart stimulating device according to claim 8, **characterized in that** the threshold search interval is decreased if the thresholds before and after any loss of capture initiated threshold search not are equal.

11. Heart stimulating device according to claim 1, **characterized in that** the threshold search interval is maintained at a duration shorter or equal than a predetermined threshold search interval, for a predetermined time period after a threshold change has occurred.

12. Heart stimulating device according to claim 11, **characterized in that** said predetermined threshold search interval is 8 hours.

13. Heart stimulating device according to claim 11, **characterized in that** said predetermined time period is longer or equal one week.
